(19) Europäisches Patentamt
European Patent Office
Office européen des brevets

(11) **EP 2 205 288 B1**

(12) **FASCICULE DE BREVET EUROPEEN**

(45) Date de publication et mention
de la délivrance du brevet:
**02.04.2014 Bulletin 2014/14**

(21) Numéro de dépôt: **07871984.6**

(22) Date de dépôt: **20.12.2007**

(51) Int Cl.:
***A61L 2/14*** *(2006.01)*     ***A61L 2/28*** *(2006.01)*
***G01N 31/22*** *(2006.01)*

(86) Numéro de dépôt international:
**PCT/FR2007/052573**

(87) Numéro de publication internationale:
**WO 2008/078053 (03.07.2008 Gazette 2008/27)**

(54) **INDICATEUR DE STERILISATION**

**STERILISATIONSINDIKATOR**

**STERILISATION INDICATOR**

(84) Etats contractants désignés:
**AT BE BG CH CY CZ DE DK EE ES FI FR GB GR HU IE IS IT LI LT LU LV MC MT NL PL PT RO SE SI SK TR**

(30) Priorité: **22.12.2006 FR 0611334**

(43) Date de publication de la demande:
**14.07.2010 Bulletin 2010/28**

(73) Titulaire: **Société pour la Conception des Applications des Techniques Electroniques - SATELEC**
**33700 Mérignac (FR)**

(72) Inventeurs:
• **RICARD, André**
**31400 Toulouse (FR)**
• **DIERAS, Françis**
**33000 Bordeaux (FR)**
• **SIXOU, Michel**
**31300 Balma (FR)**
• **VILLEGER, Sandrine**
**31380 MOntjoire (FR)**

• **CANAL, Cristina**
**08034 Barcelone (ES)**
• **ERRA, Pilar**
**08024 Barcelone (ES)**

(74) Mandataire: **Desormiere, Pierre-Louis et al Cabinet Beau de Loménie**
**158, rue de l'Université**
**75340 Paris Cedex 07 (FR)**

(56) Documents cités:
**WO-A-03/037391        US-A- 3 738 811**
**US-A- 4 188 437        US-A- 4 436 819**
**US-A- 5 340 537        US-A- 5 482 684**
**US-A1- 2001 054 374    US-A1- 2006 083 657**

• **MOREAU S ET AL: "Using the flowing afterglow of a plasma to inactivate Bacillus subtilis spores: Influence of the operating conditions" JOURNAL OF APPLIED PHYSICS, AMERICAN INSTITUTE OF PHYSICS. NEW YORK, US LNKD- DOI: 10.1063/1.373792, vol. 88, no. 2, 15 juillet 2000 (2000-07-15), pages 1166-1174, XP012051169 ISSN: 0021-8979**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

**Description**

La présente invention concerne un indicateur de stérilisation, utilisable en particulier pour la stérilisation d'instruments médicaux ou chirurgicaux, notamment à usage dentaire, à l'aide d'un plasma de post-décharge obtenu à partir d'un gaz à base d'azote et/ou d'oxygène.

La stérilisation consiste à détruire, dans une proportion donnée, un nombre significatif de microorganismes, virus ou protéines pathogènes présents à la surface, interne ou externe, des objets à traiter.

On remarquera que l'adjectif « stérile » est un terme absolu, mais l'assurance qu'un objet est stérile, c'est-à-dire exempt de microorganismes, est une fonction de probabilité. Le niveau d'assurance de stérilité SAL (Sterility Assurance Level en langue anglaise) d'un produit est défini comme la probabilité qu'une unité donnée quelconque est non stérile après l'exposition à un procédé de stérilisation validé. Ainsi, pour qu'un produit soit considéré comme stérile au regard de la norme européenne EN556, il doit posséder un niveau d'assurance de stérilité de $10^{-6}$, c'est-à-dire que la probabilité théorique d'isoler un microorganisme doit être inférieure à 1 pour $10^6$.

On connaît différents procédés de stérilisation, parmi lesquels des procédés chimiques et physiques.

Parmi les procédés chimiques de stérilisation, on peut citer les procédés faisant appel à des gaz tels que l'oxyde d'éthylène, le formaldéhyde ou le peroxyde d'hydrogène. Ces procédés présentent toutefois l'inconvénient de requérir une période importante de désorption incompatible avec une disponibilité rapide des instruments à traiter. En outre, ces gaz sont toxiques et irritent la peau et les muqueuses.

Les procédés physiques comprennent notamment la stérilisation en phase vapeur, dans un autoclave sous l'action de la température et de la vapeur ; la stérilisation à chaud par voie sèche ; la radiation par faisceaux d'ions ou rayons gamma, utilisée sur des objets qui ne peuvent être stérilisés sous l'action de la chaleur ou par voie chimique ; ou encore la filtration, à l'aide de filtres aptes à séparer les micro-organismes. Ces procédés impliquent généralement des températures élevées, souvent supérieures à 100°C.

Du fait de l'utilisation croissante de matériaux thermosensibles, comme les matériaux à base de polymères, dans les objets médicaux, il est apparu souhaitable de développer des procédés de stérilisation à basse température, et notamment à des températures inférieures à 70°C.

On a ainsi développé des procédés de stérilisation à l'aide d'un plasma, fonctionnant à des températures permettant de ne pas détériorer les matériaux thermosensibles.

On connaît par exemple de la demande WO 00/72889 un procédé de stérilisation mettant en oeuvre un plasma à base d'oxygène et d'azote. La demande FR 2 856 600 décrit quant à elle un procédé de stérilisation à l'aide d'un plasma de post-décharge émanant d'un plasma constitué exclusivement d'azote, tandis que la demande FR 2 879 933 utilise un plasma de post-décharge émanant d'un plasma constitué d'hydrogène et d'azote. De nombreux procédés de stérilisation utilisent ainsi un gaz à base d'azote ou d'oxygène pour la préparation du plasma.

Pour la validation des stérilisateurs, on utilise des indicateurs de stérilisation, qui permettent de contrôler un ou plusieurs paramètres essentiels du procédé de stérilisation. On a développé à cet effet trois types d'indicateurs de stérilisation: les indicateurs physiques, chimiques et biologiques.

Les indicateurs de stérilisation pour des procédés mettant en oeuve un plasma sont encore peu développés. On connaît des documents US 6,659,036, WO 98/46279, JP 2005111154 et JP 2004298479, des indicateurs de stérilisation relatifs à des procédés utilisant des plasmas. Toutefois, ces documents ne concernent pas des plasmas à base d'azote et/ou d'oxygène.

Les documents US-A- 5 340 537 et WO-A-03/037391 divulgent des indicateurs de stérilisation comprenant un colorant thermochromique.

Il apparaît ainsi nécessaire de disposer d'indicateurs de stérilisation, permettant d'évaluer au moins un paramètre du procédé de stérilisation, pour des procédés de stérilisation mettant en oeuvre un plasma de post-décharge obtenu à partir d'un gaz comprenant de l'azote $N_2$ et/ou de l'oxygène $O_2$.

La demanderesse a développé un indicateur de strérilisation permettant d'atteindre cet objectif. En particulier, l'indicateur selon l'invention permet d'indiquer la présence d'atomes d'oxygène O et/ou d'azote N dans un plasma de post-décharge ainsi que de vérifier la température dans la chambre de post-décharge.

L'indicateur de stérilisation selon l'invention, utilisable dans un dispositif de stérilisation, comprend :

- une première partie, comprenant un composé du type chauffant lorsqu'il est mis en contact avec des atomes d'oxygène O et/ou d'azote N, et un colorant thermochromique, en contact thermique avec le composé, et

- une deuxième partie, comprenant un colorant thermochromique, et ne comprenant pas de composé du type chauffant lorsqu'il est mis en contact avec des atomes d'oxygène O et/ou d'azote N.

Par « en contact thermique avec le composé » au sens de l'invention, on entend que le colorant est disposé par rapport au composé de façon à ce qu'un échauffement du composé provoque une élévation de température du colorant.

Le composé est de préférence sous forme de poudre, de particules, de fils ou de fibres, afin d'assurer une surface de contact élevée entre le composé et le colorant, et donc de favoriser l'échange thermique.

Le composé comprend de préférence au moins un métal. En effet, la recombinaison atomique de surface des atomes d'azote N ou d'hydrogène H, qui est exothermique, est favorisée en présence de surfaces métalliques. La probabilité de recombinaison atomique des atomes d'azote N ou d'oxygène O est en effet importante sur les surfaces métalliques. Cette réaction étant exothermique, l'énergie de réaction de la recombinaison est en partie transférée au métal.

Le métal du composé peut être choisi parmi le cuivre, le titane, les aciers, l'aluminium, et leurs alliages.

On utilise de préférence le cuivre et ses alliages.

Le colorant thermochromique peut être réversible ou irréversible. Un colorant thermochromique est un colorant qui change de couleur sous l'action de la température. Il est irréversible lorsque la couleur obtenue sous l'action de la température est permanente dans le temps et ne revient pas à la couleur d'origine.

La nature réversible ou irréversible du colorant tehrmochromique sera choisie en fonction de l'utilisation souhaitée de l'indicateur. Si l'on souhaite suivre en temps réel l'évolution de couleur du colorant et s'en servir à nouveau rapidement à l'issue de la stérilisation, on pourra employer un colorant thermochromique réversible. Si l'indicateur doit en revanche être stocké avec les appareils à stériliser en vue de la vérification ultérieure des paramètres de stérilisation, on utilisera un colorant thermochromique irréversible.

Le colorant sera choisi en fonction de la nature du composé et des paramètres du procédé de stérilisation. En effet, l'élévation de température du colorant est fonction de la capacité du composé à s'échauffer au contact des atomes d'azote N et/ou d'oxygène O, ainsi que de la quantité d'atomes d'azote N et/ou d'oxygène O avec laquelle le composé est en mis contact.

Le composé et le colorant sont de préférence en contact avec un support, c'est-à-dire disposés sur et/ou dans le support. Le composé peut ainsi être disposé ou dispersé sur un support sur lequel on a disposé le colorant. Le composé peut aussi être disposé à l'intérieur du support, par exemple par pulvérisation ou entrelacé sous forme de fibres dans le support.

On peut utiliser tout type de support, par exemple un support poreux, afin de faciliter la diffusion du colorant dans le support.

Le support peut être sous forme de plaque, par exemple en polymère ou en métal, sous forme de fibres, par exemple métalliques, naturelles ou chimiques, ou encore sous forme de matériaux, tissés ou non tissés.

Le support peut notamment être choisi parmi la cellulose, les tissus, les fibres, le coton, le papier, le papier buvard.

La deuxième partie comprend un colorant thermochromique, mais ne comprend pas de composé du type chauffant. Cette deuxième partie de l'indicateur de stérilisation permet de vérifier la température régnant dans la chambre de post-décharge (la chambre de stérilisation). En effet, le colorant n'est pas en contact avec le composé chauffant, et n'est soumis qu'à la température de la chambre de post-décharge, sans autre échauffement.

Le colorant de la deuxième partie de l'indicateur peut être le même colorant que celui présent dans la première partie.

L'invention a également pour objet un procédé d'indication de la présence d'atomes d'oxygène O et/ou d'azote N dans un plasma présent dans une chambre de post-décharge (plasma de post-décharge). Le procédé est avantageusement mis en oeuvre dans une chambre de post-décharge d'un dispositif de stérilisation utilisant un plasma obtenu à partir d'un gaz comprenant de l'oxygène $O_2$ et/ou de l'azote $N_2$. Le procédé comprend successivement :

- la mise en contact avec le plasma d'un indicateur de stérilisation selon l'invention, et

- la comparaison de la couleur du colorant de l'indicateur avec une couleur témoin.

La couleur témoin est fixée en fonction des paramètres employés lors du processus de stérilisation.

Ainsi, en comparant la couleur du colorant a l'issue du procédé avec la couleur témoin, on pourra constater si l'indicateur selon l'invention a été soumis à la quantité souhaitée d'atomes d'azote N et/ou d'oxygène O. En effet, l'échauffement du composé, et donc sa température, sont proportionnels à la quantité d'atomes d'azote N et/ou d'oxygène O avec laquelle le composé est en contact. Comme la température atteinte par le composé fixe la couleur du colorant, la couleur observée est bien liée à la quantité d'atomes d'azote N et/ou d'oxygène O avec laquelle le composé a été en contact.

La comparaison de la couleur de l'indicateur selon l'invention avec l'indicateur témoin peut être réalisée visuellement, ou à l'aide de moyens automatiques de comparaison, comme par exemple un spectrophotomètre.

Pour définir si deux couleurs sont considérées comme identiques, on peut utiliser la relation de Kubelka-Munk et la variation de couleur selon le modèle CIE Lab (modèle de représentation des couleurs développé par le CIE (Commission Internationale de l'Eclairage)).

D'après la relation de Kubelka-Munk, le rapport entre le coefficient d'absorption K du colorant et le coefficient de diffusion S du colorant est lié au coefficient de réflexion β comme suit:

$$K/S = (1-\beta)^2/(2\beta)$$

D'après le mode CIE Lab, la différence de couleur entre deux composés 1 et 2 est la suivante :

$$\Delta E^*_{ab} = ((L^*_2 - L^*_1)^2 + (a^*_2 - a^*_1)^2 + (b^*_2 - b^*_1)^2)^{1/2}$$

$$= (\Delta L^{*2} + \Delta a^{*2} + \Delta b^{*2})^{1/2}$$

relation dans laquelle L* est la luminance, a* représente la gamme de l'axe rouge->vert, et b* représente la gamme de l'axe jaune-> bleu.

Pour que les couleurs de deux colorants soient considérés comme identiques, on peut ainsi imposer que $\Delta E^*_{ab} \leq 2$.

Selon un mode de réalisation, l'indicateur peut également être utilisé pour indiquer la température dans la chambre de post-décharge. Il convient dans ce cas d'observer la couleur du colorant lorsque l'on place l'indicateur dans la chambre de post-décharge, au début du procédé de stérilisation. En effet, l'échauffement du composé sous l'effet du contact avec les atomes d'azote et/ou d'oxygène est plus lent que le changement de couleur du colorant lié à la température régnant dans la chambre de post-décharge.

On choisit de préférence dans ce mode réalisation un colorant thermochromique dont le changement de couleur est progressif lorsque la température passe de la température ambiante (20°C) à la température d'échauffement du métal, de façon à présenter au moins deux couleurs distinctes.

Un tel colorant peut notamment être un colorant chemsong ink corporation.

L'invention a ainsi pour objet un procédé d'indication a) de la présence d'atomes d'oxygène O et/ou d'azote N dans un plasma présent dans une chambre de post-décharge, notamment obtenu à partir d'un gaz comprenant de l'oxygène $O_2$ et/ou de l'azote $N_2$, et b) de la température dans la chambre de post-décharge. Le procédé comprend successivement :

- la mise en contact avec le plasma de l'indicateur de stérilisation à deux parties, et

- la comparaison des couleurs des colorants de la première et la deuxième partie de l'indicateur avec deux couleurs témoins.

On pourra comparer la couleur du colorant de la première partie de l'indicateur avec une première couleur témoin, caractéristique d'une quantité de référence d'atomes d'oxygène O et/ou d'azote N, puis on comparera la couleur du colorant de la deuxième partie de l'indicateur avec une deuxième couleur témoin, caractéristique d'une température de référence dans la chambre. On pourra également comparer les couleurs des deux parties entre elles.

Grâce à l'utilisation de l'indicateur à deux parties, il n'y a plus besoin de suivre l'évolution du procédé en temps réel.

D'autres buts, caractéristiques et avantages de l'invention ressortiront à la lecture de la description suivante, donnée uniquement à titre d'exemple, et faite en référence aux dessins annexés, sur lesquels

- la figure 1 illustre de manière schématique un dispositif de stérilisation avec un indicateur selon l'invention,
- la figure 2 montre un indicateur qui n'est pas conforme à l'invention,
- la figure 3 montre une vue de dessus d'un indicateur selon l'invention.

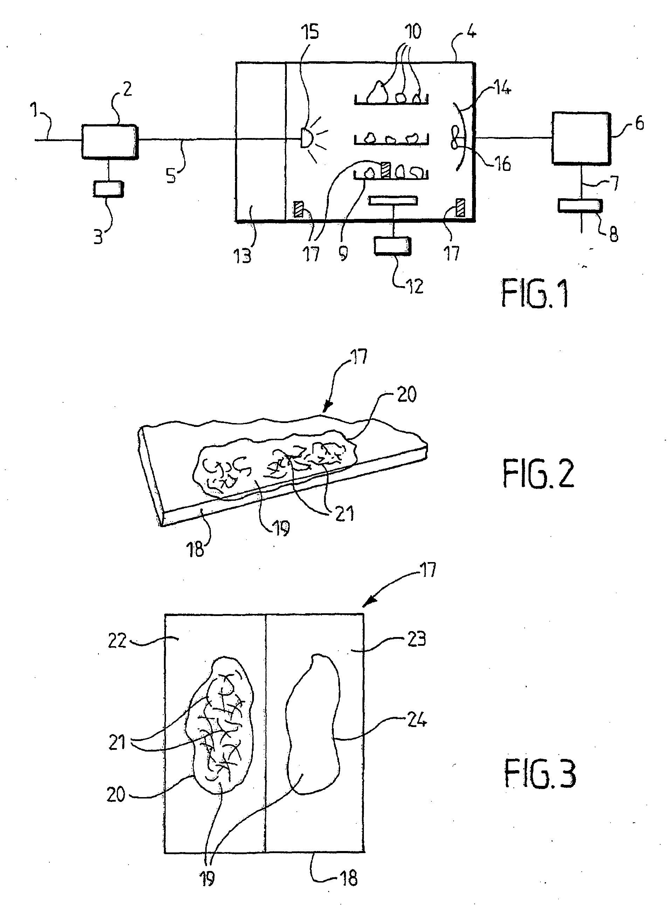

Le dispositif de stérilisation, tel qu'illustré sur la figure 1, comprend une conduite d'arrivée 1 d'un flux d'azote $N_2$ qui traverse une enceinte 2 sous vide soumise à un champ électrique produit par un générateur 3 de micro-ondes à 2,45 GHz. Le champ électrique provoque notamment la formation d'atomes N à partir des molécules $N_2$. Le plasma ainsi produit est acheminé vers une chambre de stérilisation 4 via une conduite 5, à l'aide d'une pompe à vide 6. La chambre 4 est alimentée en plasma par l'intermédiaire d'une buse 15. La buse 15 pourra avantageusement se terminer par un ou plusieurs injecteurs permettant d'homogénéiser le flux de plasma. La pompe à vide 6 assure également l'évacuation du plasma vers l'extérieur par une conduite 7 pourvue de filtres 8.

La chambre de stérilisation 4 est appelée « chambre de post-décharge », car le plasma n'y est pas soumis à un champ électrique, alors qu'il l'était dans l'enceinte 2. Le plasma présent dans la chambre 4, dit « plasma de post-décharge », non soumis à un champ électrique, ne contient ainsi plus de radiations UV, ni d'ions et d'électrons, ce qui permet d'éviter un échauffement excessif dans la chambre 4 et la détérioration des objets 10 à stériliser.

La chambre de stérilisation 4, de forme parallélépipédique, comporte un porte-instrument 9, métallique ou non métallique, qui est destiné à recevoir les objets 10 à stériliser. La chambre de stérilisation 4 est pourvue de moyens de chauffage 11 dont la température est contrôlée par un dispositif de commande 12. Ces moyens de chauffage peuvent notamment être une résistance électrique ou des moyens de chauffage par induction.

La chambre de stérilisation 4 est fermée sur l'un de ses côtés par une porte pivotante 13.

La chambre 4 peut également être pourvue d'un réflecteur 14 et d'un ventilateur 16 qui contribuent à l'homogénéisation du plasma.

On introduit dans la chambre 4 les objets 10 à stériliser et un ou plusieurs indicateurs de stérilisation 17. Les indicateurs de stérilisation 17 peuvent être disposés dans l'ensemble de la chambre 4, et notamment aux endroits difficiles d'accès, comme contre les parois ou sous les objets 10 à stériliser. De cette façon, on pourra s'assurer que tout le volume de la chambre de traitement est traité par le plasma d'azote.

Il est également particulièrement utile de disposer des indicateurs de stérilisation 17 sur les poches de stérilisation contenant les objets 10 à traiter, où à l'intérieur de celles-ci, afin de s'assurer que les objets 10 eux-mêmes ont été en contact avec les atomes d'azote N.

La pression régnant dans la chambre de stérilisation 4 est de préférence inférieure à $10^5$ Pa, afin de faciliter la mise en contact des atomes d'azote N avec les objets 10.

On a représenté sur la figure 2 un indicateur de stérilisation 17 qui nest pas conforme à l'invention.

L'indicateur de stérilisation 17 comprend un support 18, imprégné d'un colorant 19 qui a diffusé dans le support 18 pour former une zone de colorant 20. Un composé 21, sous forme de fibres métalliques, est disposé dans le support et dans la zone de colorant 20. Telles que représentées sur la figure 2, les fibres sont disposées à l'intérieur de la zone de colorant 20, mais on peut également envisager que les fibres soient dispersées dans l'ensemble du support 18, et dépassent ainsi de la zone de colorant 20.

On a représenté sur la figure 3 un indicateur de stérilisation 17 selon l'invention comprenant deux parties. L'indicateur 17 comprend une première partie 22 comprenant un colorant 19 qui a diffusé dans le support 18 pour former une zone de colorant 20. Un composé 21, sous forme de fibres métalliques, est disposé dans le support et dans la zone de colorant 20.

Dans la deuxième partie 23 de l'indicateur 17, qui ne comprend pas le composé 21, le colorant 19 forme une zone de colorant 24. Les deux zones de colorants 20 et 24 sont suffisamment éloignées pour que les fibres métalliques 21 ne soient pas en contact thermique avec la zone de colorant 24 de la deuxième partie 23.

Dans les deux exemples qui suivent, les paramètres du procédé de stérilisation sont les suivants : la température dans la chambre de stérilisation 4 est de 60°C, on met en oeuvre un débit d'azote de 1L/mn, une pression d'azote de 6.66 $10^2$ Pa (5 Torr), et une durée d'exposition au plasma de post-décharge de 40 mn.

Par ailleurs, on utilise comme colorant 19 le colorant Kromagen magenta 120 screen ink.

L'évolution de la couleur du colorant en fonction de la température est la suivante :

20°C : blanc-rose pâle

60°C : rose pâle

70°C : rose

90°C : magenta

120°C : pourpre

On a déterminé à l'aide de plusieurs essais de référence, que l'échauffement du métal des indicateurs des exemples 1 et 2 doit amener le colorant à une température de 90°C si les paramètres indiqués ci-dessus sont respectés.

Exemple 1 : Indicateur de stérilisation à une partie (pas conforme à l'invention)

Le support 18 est constitué de fibres méta-aramides.

Le composé 21 est constitué de fils d'alliage cuivre-nickel de diamètre 20 $\mu$m, commercialisé sous la dénomi-

nation Monel® par la société Baltec ltd. Un mode de fabrication de l'indicateur consiste à mettre en contact les fils 21 avec le support 18, puis à verser le colorant 19 sur le support 18, et à laisser sécher à 20°C. L'indicateur 17 ainsi fabriqué présente une couleur blanche/rose pâle.

L'indicateur 17 est placé dans la chambre 4 pour être soumis au procédé de stérilisation.

On observe le colorant 19 de l'indicateur 17 au bout d'une minute de stérilisation. Il est de couleur rose pâle, ce qui montre que la température dans la chambre 4 est sensiblement de 60°C.

A l'issue du procédé, l'indicateur 17 est de couleur magenta (rose sombre), qui est la couleur souhaitée.

Exemple 2 : Indicateur de stérilisation à deux parties

Le support 18 est constitué de fibres de coton.

Un mode de fabrication de l'indicateur consiste à mélanger des fils de cuivre de diamètre 20 $\mu$m avec des fibres de coton dans la première partie 22 de l'indicateur 17, puis à étendre le colorant 19 sur le support 18, à la fois dans la première partie 22 et dans la deuxième partie 23, de façon à former deux zones de colorant 20 et 24, et à laisser sécher à 20°C. L'indicateur 17 ainsi fabriqué présente une couleur blanche/rose pâle.

A l'issue du procédé, la zone de colorant 24 est rose pâle, ce qui montre que la température de la chambre 4 est sensiblement de 60°C. La zone de colorant 20 est couleur magenta (rose sombre). Le test est donc validé.

Ainsi, la détermination de la couleur du colorant en contact avec le métal est effectuée en prenant en compte les différents paramètres que l'on souhaite utiliser lors du procédé de stérilisation, comme la concentration en atomes d'azote N dans le plasma, la durée de l'exposition des objets 10 au plasma, la température régnant dans la chambre de stérilisation 4, et le volume des objets 10. La concentration en atomes d'azote N pourra être choisie en réglant la puissance du générateur 3 de micro-ondes ainsi que le débit d'azote.

La détermination de la couleur du colorant prend également en compte la nature du métal 21, puisque l'échauffement de la surface métallique est fonction de la nature du métal, en plus de la quantité d'atomes d'azote N en contact avec le métal 21.

Ainsi, grâce à l'indicateur 17 selon l'invention, on peut valider deux paramètres du procédé de stérilisation qui sont la quantité d'atomes d'azote N et la température de stérilisation.

On peut décider de déterminer le niveau d'assurance de stérilité du procédé en fonction de la pression et du débit d'azote, de la teneur en atomes d'azotes N, de la température dans la chambre 4 de stérilisation, et de la durée de stérilisation. Comme la densité en atomes d'azote N est proportionnelle à la puissance du générateur 3 de micro-ondes, à la pression et au débit d'azote, les paramètres à vérifier seront la température, la teneur en atomes d'azotes N et la durée du traitement.

L'indicateur de stérilisation 17 selon l'invention est donc particulièrement adapté pour s'assurer que les atomes d'azote N présents dans le plasma de post-décharge ont été en contact avec l'ensemble de la chambre de stérilisation, ainsi qu'avec les objets 10 à stériliser. L'indicateur 17 fonctionne également avec des plasmas de post-décharge obtenus à partir de gaz comprenant un mélange d'azote $N_2$ avec d'autres espèces, comme les plasmas obtenus à partir de $N_2/H_2$, ou $Ar/N_2$.

L'indicateur 17 fonctionne également avec les atomes d'oxygène O présent dans un plasma de post-décharge obtenu à partir d'un gaz comprenant de l'oxygène $O_2$, comme les plasmas obtenus à partir de $N_2/O_2$ ou $Ar/O_2$.

**Revendications**

1. Indicateur de stérilisation (17) comprenant une première partie (22), comprenant un composé (21) du type chauffant lorsqu'il est mis en contact avec des atomes d'oxygène O et/ou d'azote N, et un colorant thermochromique (19), en contact thermique avec le composé (21),
   **caractérisé en ce qu'**il comprend en outre une deuxième partie (23), comprenant un colorant thermochromique (19), et ne comprenant pas de composé (21) du type chauffant lorsqu'il est mis en contact avec des atomes d'oxygène O et/ou d'azote N.

2. Indicateur (17) selon la revendication 1, **caractérisé en ce que** le composé (21) comprend au moins un métal.

3. Indicateur (17) selon la revendication 2, **caractérisé en ce que** le métal est choisi parmi le cuivre, le titane, les aciers, l'aluminium et leurs alliages.

4. Indicateur (17) selon la revendication 3, **caractérisé en ce que** le métal est choisi parmi le cuivre et ses alliages.

5. Indicateur (17) selon l'une des revendications 1 à 4, **caractérisé en ce que** le composé (21) est sous forme de poudre, de particules, de fils ou de fibres.

**6.** Indicateur (17) selon l'une des revendications 1 à 5, **caractérisé en ce que** le composé (21) et le colorant (19) sont en contact avec un support (18).

**7.** Indicateur (17) selon la revendication 6, **caractérisé en ce que** le support (18) est sous forme d'une plaque ou sous forme de fibres.

**8.** Indicateur (17) selon la revendication 7, **caractérisé en ce que** le support (18) est choisi parmi la cellulose, les tissus, le coton, le papier, le papier buvard.

**9.** Indicateur (17) selon l'une des revendications 1 à 8, **caractérisé en ce que** le colorant (19) est un colorant thermochromique irréversible.

**10.** Utilisation d'un indicateur (17) selon l'une des revendications 1 à 9 dans un dispositif de stérilisation.

**11.** Utilisation selon la revendication 10, pour l'indication de la présence d'atomes d'oxygène O et/ou d'azote N dans un plasma de post-décharge.

**12.** Utilisation selon la revendication 11, pour indiquer en outre la température dans une chambre (4) de post-décharge.

**13.** Procédé d'indication de la présence d'atomes d'oxygène O et/ou d'azote N dans un plasma présent dans une chambre (4) de post-décharge, **caractérisé en ce qu'**il comprend successivement :

- la mise en contact avec le plasma d'un indicateur de stérilisation (17) selon l'une des revendications 1 à 9, et
- la comparaison de la couleur du colorant (19) de la première partie de l'indicateur (17) avec une couleur témoin.

**14.** Procédé selon la revendication 13 d'indication en outre en outre de la température dans la chambre de post-décharge, **caractérisé en ce qu'** il comprend

- la comparaison des couleurs des colorants (19) de la première (22) et la deuxième partie (23) de l'indicateur (17) avec deux couleurs témoins.


**Patentansprüche**

**1.** Sterilisationsindikator (17), umfassend einen ersten Teil (22), der eine Verbindung (21) vom sich erwärmenden Typ, wenn sie mit Sauerstoff O- und/oder Stickstoff N-Atomen in Kontakt gebracht wird, und einen thermochromen Farbstoff (19), der mit der Verbindung (21) in Wärmekontakt ist, umfasst, **dadurch gekennzeichnet, dass** er ferner einen zweiten Teil (23) umfasst, der einen thermochromen Farbstoff (19) umfasst und keine Verbindung (21) vom sich erwärmenden Typ, wenn sie mit Sauerstoff O- und/oder Stickstoff N-Atomen in Kontakt gebracht wird, umfasst.

**2.** Indikator (17) nach Anspruch 1, **dadurch gekennzeichnet, dass** die Verbindung (21) wenigstens ein Metall umfasst.

**3.** Indikator (17) nach Anspruch 2, **dadurch gekennzeichnet, dass** das Metall aus Kupfer, Titan, Stählen, Aluminium und deren Legierungen ausgewählt ist.

**4.** Indikator (17) nach Anspruch 3, **dadurch gekennzeichnet, dass** das Metall aus Kupfer und seinen Legierungen ausgewählt ist.

**5.** Indikator (17) nach einem der Ansprüche 1 bis 4, **dadurch gekennzeichnet, dass** die Verbindung (21) in Form von Pulver, von Partikeln, von Fäden oder von Fasern vorliegt.

**6.** Indikator (17) nach einem der Ansprüche 1 bis 5, **dadurch gekennzeichnet, dass** die Verbindung (21) und der Farbstoff (19) mit einem Träger (18) in Kontakt sind.

**7.** Indikator (17) nach Anspruch 6, **dadurch gekennzeichnet, dass** der Träger (18) in Form einer Platte oder in Form von Fasern vorliegt.

8. Indikator (17) nach Anspruch 7, **dadurch gekennzeichnet, dass** der Träger (18) aus Zellulose, Geweben, Baumwolle, Papier, Löschpapier ausgewählt ist.

9. Indikator (17) nach einem der Ansprüche 1 bis 8, **dadurch gekennzeichnet, dass** der Farbstoff (19) ein irreversibler thermochromer Farbstoff ist.

10. Verwendung eines Indikators (17) nach einem der Ansprüche 1 bis 9, in einer Sterilisationsvorrichtung.

11. Verwendung nach Anspruch 10, für das Anzeigen des Vorhandenseins von Sauerstoff O- und/oder Stickstoff N-Atomen in einem Nachentladungsplasma.

12. Verwendung nach Anspruch 11, um ferner die Temperatur in einer Nachentladungskammer (4) anzuzeigen.

13. Verfahren zum Anzeigen des Vorhandenseins von Sauerstoff O- und/oder Stickstoff N-Atomen in einem in einer Nachentladungskammer (4) vorhandenen Plasma, **dadurch gekennzeichnet, dass** es nacheinander umfasst:

  - das Inkontaktbringen eines Sterilisationsindikators (17) nach einem der Ansprüche 1 bis 9, mit dem Plasma, und
  - das Vergleichen der Farbe des Farbstoffes (19) des ersten Teils des Indikators (17) mit einer Kontrollfarbe.

14. Verfahren nach Anspruch 13, ferner zum Anzeigen der Temperatur in der Nachentladungskammer, **dadurch gekennzeichnet, dass** es umfasst

  - das Vergleichen der Farben der Farbstoffe (19) des ersten (22) und zweiten Teils (23) des Indikators (17) mit zwei Kontrollfarben.


**Claims**

1. A sterilization indicator (17), comprising a first portion (22) comprising a compound (21) of the heating type on being put into contact with atoms of oxygen O and/or nitrogen N, and a thermochromic dye (19) in thermal contact with the compound (21);
**characterized in that** it further comprises a second portion (23), comprising a thermochromic dye (19), and not including a compound (21) of the heating type when put into contact with atoms of oxygen O and/or nitrogen N.

2. An indicator (17) according to claim 1, **characterized in that** the compound (21) comprises at least one metal.

3. An indicator (17) according to claim 2, **characterized in that** the metal is selected from copper, titanium, steels, aluminum, and alloys thereof.

4. An indicator (17) according to claim 3, **characterized in that** the metal is selected from copper and alloys thereof.

5. An indicator (17) according to any one of claims 1 to 4, **characterized in that** the compound (21) is in the form of a powder, particles, filaments, or fibers.

6. An indicator (17) according to any one of claims 1 to 5, **characterized in that** the compound (21) and the dye (19) are in contact with a medium (18).

7. An indicator (17) according to claim 6, **characterized in that** the medium (18) is in the form of a plate or in the form of fibers.

8. An indicator (17) according to claim 7, **characterized in that** the medium (18) is selected from cellulose, fabrics, cotton, paper, and blotting paper.

9. An indicator (17) according to any one of claims 1 to 8, **characterized in that** the dye (19) is an irreversible thermochromic dye.

**10.** The use of an indicator (17) according to any one of claims 1 to 9, in a sterilization device.

**11.** A use according to claim 10, for indicating the presence of atoms of oxygen O and/or nitrogen N in a post-discharge plasma.

**12.** The use of an indicator (17) according to claim 11 for indicating in addition the temperature in a post-discharge chamber (4).

**13.** A method of indicating the presence of atoms of oxygen O and/or nitrogen N in a plasma present in a post-discharge chamber (4), the method being **characterized in that** it comprises in succession:

   • putting a sterilization indicator (17) according to any one of claims 1 to 9 into contact with the plasma; and
   • comparing the color of the dye (19) of the first portion of the indicator (17) with a reference color.

**14.** A method according to claim 13 of indicating the temperature in the post-discharge chamber,
the method being **characterized in that** it comprises comparing the colors of the dyes (19) of the first and second portions (22, 23) of the indicator (17) with two reference colors.

FIG.1

FIG.2

FIG.3

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 0072889 A
- FR 2856600
- FR 2879933
- US 6659036 B
- WO 9846279 A
- JP 2005111154 B
- JP 2004298479 B
- US 5340537 A
- WO 03037391 A